# EUROPÄISCHE PATENTSCHRIFT

(11) **EP 1 637 584 B1**
(45) Veröffentlichungstag und Bekanntmachung des Hinweises auf die Patenterteilung: **11.07.2007**
(21) Anmeldenummer: 05011858.7
(22) Anmeldetag: 01.06.2005
(51) Int. Cl.: C12M 1/00, C12M 1/28, B01L 3/00

(54) **Beutel mit darin gezüchteter Kultur sowie Probeentnahmeverfahren**
Culture bag and method for sample collection
Sac de culture et procédé de prélèvement d'echantillon

(30) Priorität: 20.09.2004 DE 102004045916; 21.02.2005 DE 102005008144
(43) Veröffentlichungstag der Anmeldung: 22.03.2006
(73) Patentinhaber: Infors AG, 4103 Bottmingen (CH)
(72) Erfinder: Hawrylenko, Alexander, 4103 Bottmingen (CH)
(74) Vertreter: Säger, Manfred

(56) Entgegenhaltungen:
- DE-A1- 19 608 110
- US-A- 4 910 147

## Beschreibung

Die Erfindung betrifft einen Beutel gemäss dem Oberbegriff des Hauptanspruchs, nämlich einen als Reaktor für eine Kultur dienender Beutel, dessen Inneres nach dem Impfen eine Kultur zur Züchtung derselben in Suspension (flüssiges Medium) oder auf einem Carrier für die Kultur in Form von Kügelchen, Gitter, Netzen od.dgl. aufweist sowie desweiterern ein Verfahren zur Entnahme einer Probe der in dem Beutel nach einem der Sachansprüche gezüchteten Kultur.

Solche Beutel sind bekannt. Sie dienen nach dem Impfen zur Züchtung einer Kultur, für die sie entweder eine Suspension in Form einern Nährflüssigkeit oder auch einen Carrier wie Kügelchen, Gitter, Netzen od. dgl. aufweisen. Ein Kulturbeutel mit einer trennbaren Einfullöffnung ist aus US-A-4910147 bekannt.

Von Nachteil bei diesen bekannten, als Reaktoren dienenden Beuteln ist die Tatsache, dass derartige Beutel nach bereits einmaliger Entnahme (z.B. einer Probe) nicht mehr steril sind. Es ist damit nur eine einzige Ernte möglich.

Der Erfindung liegt die Aufgabe zugrunde, einen gattungsgemäßen Beutel so auszugestalten, daß eine Vielzahl von sterilen Entnahmen unter gleichzeitigem Erhalt der Sterilität des Beutelinneren möglich ist.

Diese Aufgabe wird bei einem gattungsgemässen Beutel nach dem Oberbegriff des Sach- und Verfahrenshauptanspruchs erfindungsgemäss durch dessen kennzeichnende Merkmale also bei einem gattungsgemässen Beutel dadurch gelöst, dass der Beutel einen Fingerbereich mit zumindest zwei fingerartigen, in das Innere des Beutels hineinragenden Einstülpungen aufweist, die auf die Aussenseite des Fingerbereichs des Beutels als Ausstülpung herausstülpbar sind, und dass der Werkstoff dieses Fingerbereichs des Beutels siegel- und/oder schweissbar ist, und bei dem Verfahren dadurch gelöst, dadurch gekennzeichnet, dass die fingerartige, in das Innere des Fingerbereichs des Beutels hineinragende Einstülpung zum Entnehmen eines Teils der Kultur auf die Aussenseite des Beutels als fingerartige Ausstülpung herausstülpbar ist, dass diese Ausstülpung an ihrem Grund mittels zweier voneinander über einen zwischen der Ausstülpung und dem Rest des Fingerbereichs des Beutels befindlichen Zwischenbereich beabstandeter Dichtbereiche dicht entweder abgesiegelt oder abgeschweisst werden und dass danach der Zwischenbereich von dem Rest des Beutels durch Schneiden, Abscheren od. dgl. separiert wird.

Zu Beginn der Züchtung der Kultur sind die fingerartigen Einstülpungen im Inneren des Beutels angeordnet. Die Kultur wächst zunächst entweder in dem im Beutelinneren befindlichen Medium oder auf der Oberfläche des Carriers sowohl in dem dafür vorgesehenen Beutelteil als auch auf der Oberfläche der jeweiligen Einstülpung. Wenn eine Entnahme erfolgen soll, wird einer der fingerartigen Einstülpungen beispielsweise durch Vakuum oder mittels einer Handhabe als Ausstülpung nach aussen gestülpt und an ihrem Grund doppelt unter Belassung eines Zwischenbereichs abgesiegelt oder abgeschweisst und danach von dem Beutel im Zwischenbereich separiert (z.B. durch Absiegelung oder Abschneiden). Dadurch wird es möglich, den abgetrennten Fingerbereich in Form der Ausstülpung mit der dabei entnommenen Kultur zu untersuchen, ohne daß das Innere des Beutels unsteril wird.

Zweckmäßige Ausgestaltungen und Weiterbildungen sind in den Unteransprüchen gekennzeichnet.

Ein bevorzugtes Ausführungsbeispiel der Erfindung wird nachfolgend unter Bezugnahme auf die Zeichnung näher erläutert. In dieser zeigt:
- Figur 1: eine erste Ausführungsform eines erfindungsgemässen Beutels mit einer Kultur in Suspension, in schematischer Draufsicht;
- Figur 2: eine zweite Ausführungsform eines erfindungsgemässen Beutels, in schematischer Draufsicht;
- Figur 3: den Beutel gemäss Figur 1, mit einer nach aussen gestülpten Ausstülpung im doppelt abgesiegelten Zustand
- Figur 4: den Beutel gemäss Figur 1, mit einer Kultur auf einem Carrier, in schematischer Draufsicht
- Figur 5: den Beutel gemäss Figur 4, mit einer nach aussen gestülpten Ausstülpung und
- Figur 6: den Beutel gemäss Figur 5, mit der nach aussen gestülpten Ausstülpung im doppelt abgesiegelten Zustand

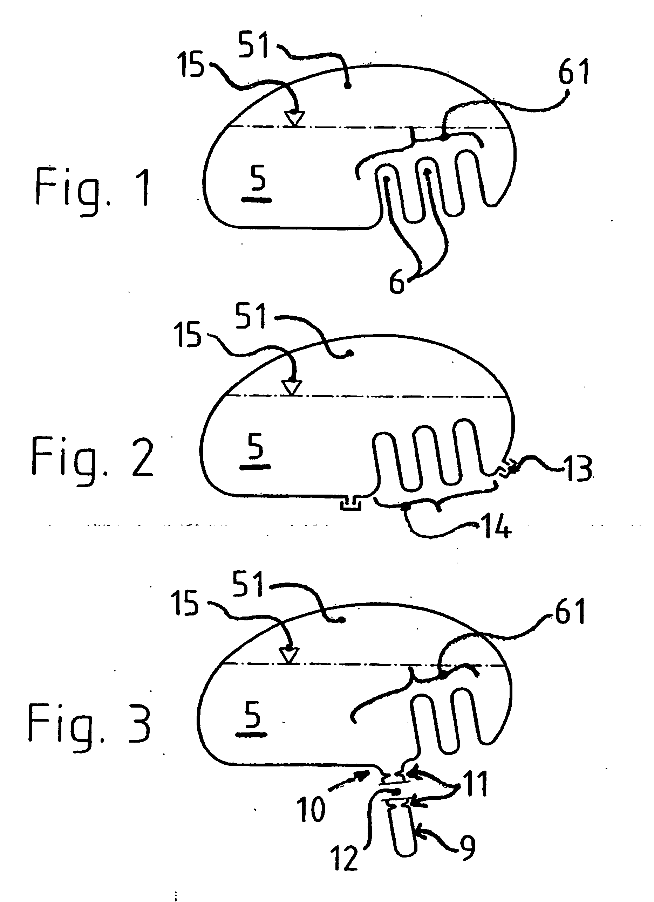

Die erste Ausführungsform (Figur 1) eines insgesamt mit 5 bezeichneten Beutels aus einem siegel- und/oder schweissbaren Werkstoff weist einen Fingerbereich 61 mit einer Vielzahl von in sein Inneres 51 ragenden -eingestülpten- insgesamt mit 6 bezeichneten Einstülpungen auf und ist mit einem Medium ind der in Suspension 15 befindlichen Kultur gefüllt. Die dazu alternative Ausführungsform (Figur 2) weist einen gesonderten Fingerbereich 14 mit einem flanschartigen Randbereich auf, der an den ebenfalls flanschartigen Rand des restlichen Beutels in der Form angepasst und mit diesem mittels einer klammerartigen Verbindung verbunden ist.

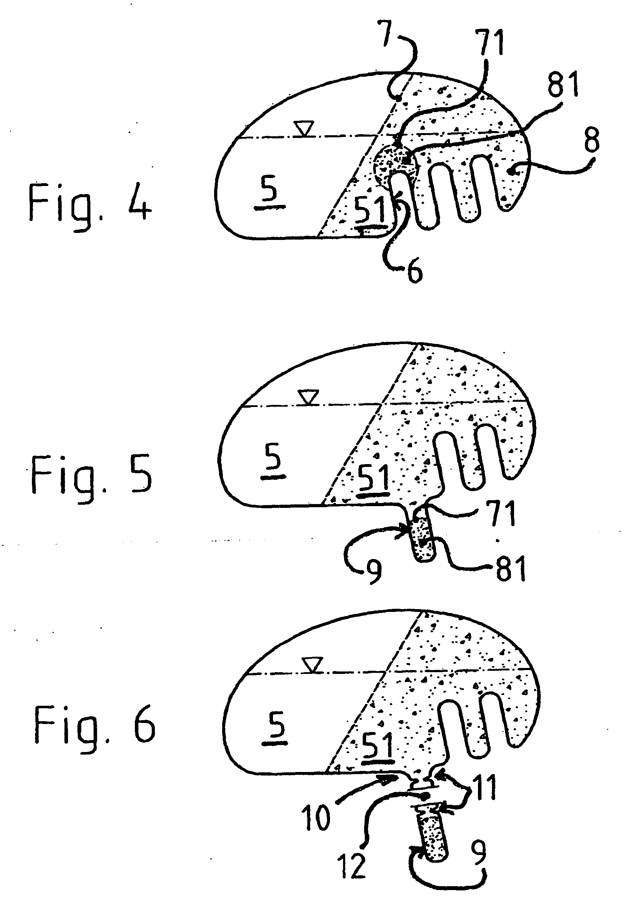

Statt der Suspension 15 kann im Inneren 51 des Beutels 5, wie in Figur 4 gezeigt, ein insgesamt mit 7 bezeichneter Teilbereich vorgesehen sein, in welchem ein insgesamt mit 8 bezeichneter Carrier, zum Beispiel in Form von Kügelchen oder Gittern bzw. Netzen zur Anlagerung der geimpften Kultur angeordnet ist. Auf dieselbe Art wird ein Teilbereich 71 mit dem gleichen Carrier 81 an der Spitze der Einstülpung 6 des Beutels 5 festgelegt.

Die Einstülpung 6 kann beispielsweise mittels Vakuum oder mittels einer Handhabe von der in Figur 2 oder 4 gezeigten, nach innen eingestülpten Lage nach aussen unter Bildung einer Ausstülpung 9, wie in Figur 3 und 5 gezeigt, gestülpt werden. Hierbei wird das im Innern angebrachte Netz in dem Teilbereich 71 mit der Carrier 81 nunmehr in die Spitze der Ausstülpung 9 bewegt (Figur 5), so dass nunmehr mit der Entnahme einer Probe begonnen werden kann. Bei der Ausführungsform mit der Suspension (Figur 3) ist es dabei nur notwendig, dass sich der Fingerbereich 61 unten befindet, so dass sich auch in der Ausstülpung 9 Kultur in Suspension befindet.

Die Ausstülpung 9 wird dann, wie in den Figuren 3 und 6 gezeigt, an ihrem Grund 10 mittels zweier voneinander beabstandeter Dichtbereiche 11 abgesiegelt oder abgeschweisst unter Belassung eines dazwischen angeordneten Zwischenbereiches 12. Mit diesen beiden Dichtbereichen 11 wird zweierlei erreicht. Einerseits ist mit dem -in der Zeichnung unteren- ersten Dichtbereich 11 das Innere der Ausstülpung 9 nebst darin befindlicher Probe vom Bereich deren Grundes 10 abgedichtet, andererseits ist mit dem -in der Zeichnung oberen- zweiten Dichtbereich 11 der restliche Beutel 5 nebst seiner Kultur von der Ausstülpung 9 ebenfalls steril abgedichtet. Nunmehr kann der Zwischenbereich 12 durch Schneiden, Abscheren oder auch Abschweissen oder Absiegeln von dem Fingerbereich 61 des Beutels 5 separiert werden, und zwar ohne, dass durch diese Probeentnahme der in der Ausstülpung 9 befindlichen Kultur weder diese noch das Innere des Beutels mit der restlichen Kultur unsteril wird.

## Patentansprüche

1. Als Reaktor für eine Kultur dienender Beutel (5), dessen Inneres nach dem Impfen eine Kultur zur Züchtung derselben in Suspension oder auf zumindest einem Carrier (8) für die Kultur in Form von Kügelchen, Gitter, Netzen od. dgl. aufweist, **dadurch gekennzeichnet, dass** der Beutel (5) einen Fingerbereich mit zumindest zwei fingerartigen, in das Innere des Beutels (5) hineinragenden Einstülpungen (6) aufweist, die auf die Aussenseite des Fingerbereichs des Beutels (5) als Ausstülpung (9) herausstülpbar sind, und dass der Werkstoff dieses Fingerbereichs des Beutels (5) siegel- und/oder schweissbar ist.

2. Beutel nach Abspruch 1, **dadurch gekennzeichnet, dass** auf der Spitze der auf die Aussenseite des Fingerbereichs des Beutels (5) herausgewölbten, fingerartigen Ausstülpung (9) eine Handhabe angeformt ist.

3. Beutel nach Anspruch 2, **dadurch gekennzeichnet, dass** die Handhabe als Ring ausgebildet ist.

4. Beutel nach einem der Ansprüche 1 bis 3, **dadurch gekennzeichnet, dass** im Fingerbereich der in das Innere (51) des Beutels (5) hineinragenden Einstülpungen (6) ein Carrier (81) für die Kultur festgelegt ist.

5. Beutel nach Anspruch 4, **dadurch gekennzeichnet, dass** der Carrier (81) in einem Netz (71) od. dgl. angeordnet ist, das seinerseits an der in das Innere (51) des Fingerbereichs des Beutels (5) hineinragenden Einstülpungen (6) festgelegt ist.

6. Verfahren zur Entnahme einer Probe der in einem Beutel nach einem der. Ansprüche 1 bis 5 gezüchteten Kultur, **dadurch gekennzeichnet, dass** die fingerartige, in das Innere (51) des Fingerbereichs des Beutels (5) hineinragende Einstülpung (6) zum Entnehmen eines Teils der Kultur auf die Aussenseite des Beutels als fingerartige Ausstülpung (9) herausstülpbar ist, dass diese Ausstülpung. (9) an ihrem Grund (10) mittels zweier voneinander über einen zwischen der Ausstülpung (9) und dem Rest des Fingerbereichs des Beutels (5) befindlichen Zwischenbereich (12) beabstandeter Dichtbereiche (11) dicht entweder abgesiegelt oder abgeschweisst werden und dass danach der Zwischenbereich (12) von dem Rest des Beutels (5) durch Schneiden, Abscheren od. dgl. separiert wird.

## Claims

1. Bag (5), used as a reactor for a culture, of which the interior comprises a culture for the cultivation thereof in suspension or on at least one carrier (8) for the culture after inoculation in the form of beads, mesh, nets or similar
**characterised in that**
the bag (5) provides a finger region with at least two finger-like internal projections (6) projecting into the interior of the bag (5), which can be folded out to the exterior of the finger region of the bag (5) as external projections, and that the material of this finger region of the bag (5) is capable of being sealed and/or welded.

2. Bag according to claim 1,
**characterised in that**
a hand-grip is formed at the tip of the finger-like external projection (9) folded out to the exterior of the finger region of the bag (5).

3. Bag according to claim 2,
**characterised in that**
the hand-grip is formed as a ring.

4. Bag according to any one of claims 1 to 3,
**characterised in that**
a carrier (81) for the culture is attached in the finger region of the internal projections (6) projecting into the interior (51) of the bag (5).

5. Bag according to claim 4,
**characterised in that**
the carrier (81) is disposed in a net (71) or similar, which, for its part, is attached to the internal projections (6) projecting into the interior (51) of the finger region of the bag (5).

6. Method for the removal of a sample of the culture cultivated in a bag according to any one of claims 1 to 5,
**characterised in that**
the finger-like internal projection (6) projecting into the interior (51) of the finger region of the bag (5) can be folded out to the exterior of the bag as a finger-like external projection (9) in order to remove a portion of the culture, that this external projection (9) is sealed off or welded in a sealing manner at its base (10) by means of two sealing regions (11) distanced from one another by an intermediate region (12) and disposed between the external projection (9) and the remainder of the finger region of the bag (5), and that the intermediate region (12) is then separated from the remainder of the bag (5) by cutting, tearing or similar.

## Revendications

1. Poche (5) servant de réacteur pour une culture, dont l'intérieur, après l'inoculation présente une culture afin de cultiver celle-ci en suspension ou sur au moins un support (8) destiné à la culture sous la forme de billes, une grille, un treillis ou similaire, **caractérisée en ce que** la poche (5) présente une zone pour les doigts comportant au moins deux renfoncements (6) ayant la forme des doigts, faisant saillie vers l'intérieur de la poche (5), qui peuvent ressortir sur la face externe de la zone pour les doigts de la poche (5) sous la forme d'une protubérance (9), et **en ce que** la matière de cette zone pour les doigts de la poche (5) peut être scellée et/ou soudée.

2. Poche selon la revendication 1, **caractérisée en ce que**, au sommet de la protubérance (9) ayant la forme de doigt, incurvée vers la face externe de la zone pour les doigts de la poche (5), est formé un élément de préhension.

3. Poche selon la revendication 2, **caractérisée en ce que** l'élément de préhension est configuré sous la forme d'une bague.

4. Poche selon l'une quelconque des revendications 1 à 3, **caractérisée en ce que**, dans la zone pour les doigts des renfoncements (6) en saillie vers l'intérieur (51) de la poche (5), est fixé un support (81) destiné à la culture.

5. Poche selon la revendication 4, **caractérisée en ce que** le support (81) est disposé dans un treillis (71) ou similaire, qui est pour sa part fixé sur les renfoncements (6) en saillie vers l'intérieur (51) de la zone pour les doigts de la poche (5).

6. Procédé permettant de prélever un échantillon d'une culture cultivée dans une poche selon l'une quelconque des revendications 1 à 5, **caractérisé en ce que** le renfoncement (6) en forme de doigt, faisant saillie vers l'intérieur (51) de la zone pour les doigts de la poche (5), peut ressortir, afin de prélever une partie de la culture, sur la face externe de la poche sous la forme d'une protubérance (9) de type doigt, **en ce que** cette protubérance (9), au niveau de son fond (10), peut être décollée ou dessoudée de manière hermétique à l'aide de deux zones d'étanchéité (11) éloignées l'une de l'autre en passant par une zone intermédiaire (12) se trouvant entre la protubérance (9) et le reste de la zone pour les doigts de la poche (5), et **en ce que**, la zone intermédiaire (12) est alors séparée du reste de la poche (5) par découpe, cisaillement, ou similaire.
